# EUROPÄISCHE PATENTSCHRIFT

(11) **EP 0 726 274 B1**
(45) Veröffentlichungstag und Bekanntmachung des Hinweises auf die Patenterteilung: **10.10.2001**
(21) Anmeldenummer: 96101018.8
(22) Anmeldetag: 25.01.1996
(51) Int. Cl.: C07H 21/00, A61K 31/70

(54) **G-Cap stabilisierte Oligonucleotide**
G-cap stabilized oligonucleotides
Oligonucléotides stabilisés par des radicaux G-gap

(30) Priorität: 31.01.1995 DE 19502912
(43) Veröffentlichungstag der Anmeldung: 14.08.1996
(73) Patentinhaber: Aventis Pharma Deutschland GmbH, 65929 Frankfurt am Main (DE)
(72) Erfinder: Peyman, Anuschirwan, Dr., D-65779 Kelkheim (DE); Uhlmann, Eugen, Dr., D-61479 Glashütten (DE)

(56) Entgegenhaltungen:
- WO-A-92/20348
- WO-A-94/02499
- WO-A-95/01363
- D. JAYASENA ET AL.: "Modified RNA sequence pools for in vitro selection" NUCLEIC ACIDS RES., Bd. 22, 1994, Seiten 5229-34, XP002043116
- R.F. MACAYA ET AL.: "Thrombin-binding aptamer forms a unimolecular quadruplex structure in solution" PROC. NATL. ACAD. SCI. USA, Bd. 90, 1993, Seiten 3745-9, XP002043117
- R. CECH ET AL.: "Monovalent cation-induced structure of telomeric DNA: the G-quartet model" CELL, Bd. 59, 1989, Seiten 87871-80, XP002043118

## Beschreibung

Antisense Oligonucleotide (AO), Tripel Helix Bildende Oligonucleotide (TFO) und sense Oligonucleotide erwiesen sich als spezifische Inhibitoren der Genexpression in einer Vielzahl von Systemen, sowohl in vitro als auch in vivo [Uhlmann & Peyman, Chem. Rev. 1990, 90, 543; Milligan et al., J. Med. Chem. 1993, 36, 1923; Stein & Cheng, Science 1993, 261, 1004; Bielinski et al., Science 1990, 250, 997].

Eines der Hauptprobleme bei der Verwendung natürlich vorkommender Phosphodiester (PO) Oligonucleotide ist deren rascher Abbau durch verschiedene nucleolytische Aktivitäten sowohl in Zellen, wie auch im Zellkultur-Medium. Zur Stabilisierung von Oligonucleotiden wurden verschiedene chemische Modifikationen eingesetzt. Eine Übersicht über den Stand der Technik geben beispielsweise Uhlmann & Peyman, supra und P.D. Cook [Antisense Research and Applications, Crooke und Lebleu, Eds., Chapter 9, S. 149ff, CRC Press Boca Raton 1993]. Die Stabilisierung gegen nucleolytischen Abbau kann durch die Modifikation oder Ersatz der Phosphatbrücke, des Zuckerbausteins, der Nucleobase oder durch Ersatz des Zucker-Phosphat-Rückgrats der Oligonucleotide erfolgen. Da die Phosphatbrücke das Zentrum des nucleolytischen Angriffs ist, wurden insbesondere zahlreiche Modifikationen der Internucleosidbrücke beschrieben. Die am häufigsten verwendeten nucleaseresistenten Internucleosidbrücken sind Phosphorothioat-(PS), Methylphosphonat- (MeP) und Phosphoroamidat- (PA) Brücken. Eine weitere Möglichkeit der Stabilisierung sind "hairpin" oder "self-stabilized" Oligonucleotide wie sie z.B. bei Tang et al. [Nucl. Acids Res. 21: 2729, 1993] beschrieben werden.

Ein weiteres Problem der Antisense Technologie ist die oft unzureichende Zellpenetration der Oligonucleotide. Zur Verbesserung wurden auch hier zahlreiche chemische Modifikationen eigesetzt. Eine Übersicht über den Stand der Technik geben Uhlmann & Peyman, supra und P.D. Cook, supra. Diese Modifikationen beinhalten u.a. lipophile Konjugat-Gruppen am **5'**- oder **3'**-Ende der Oligonucleotide und neutrale oder ionische "backbone"-Modifikationen sowie 2'-Modifikationen am Pentofuranosylring. Hughes [Antisense Research and Development **4: 211**, **1994**] zeigt, daß die Zell-Aufnahme von einem **10**-meren Homo-G Phosphorothioat etwa um den Faktor 2 höher ist als die Zell-Aufnahme der **10**-meren Homo-Oligomeren Phosphorothioate von T, A, oder C.

Blackburne [Nature **350: 569, 1991**] beschreibt Strukturen, die von G-reichen Oligonucleotiden eingenommen werden können. G-reiche Oligonucleotide mit mindestens vier kurzen Abschnitten mit G-Resten sind in der Lage, in Gegenwart von Na⁺ oder K⁺ kompakte intramolekulare Strukturen zu bilden, die als stabilisierendes Element sogenannte G-Quartette enthalten, dies sind vier in einer Ebene über Hoogsten Basenpaarung verknüpfte Guanin-Reste. Mehrere solche Elemente sind übereinander angeordnet. Oft sind Oligonucleotide zu kurz, um solche intramolekularen Strukturen auszubilden. In solchen Fällen können G-Quartett Strukturen durch Assoziation zweier Oligonucleotide gebildet werden.

Es wurde nun gefunden, daß es eine sehr einfache Möglichkeit gibt, unmodifizierte oder modifizierte Oligonukleotide hinsichtlich ihrer Nuclease-Resistenz und Zellpenetration deutlich zu verbessern nämlich durch Verlängerung der Oligonucleotide am **3'**- und/oder **5'**-Ende um ein bis **10** Guanine, so daß die Wirksamkeit erheblich verbessert wird.

Überraschenderweise zeigen auch die erfindungsgemäßen Oligonucleotide eine Tendenz zur Assoziation bzw. Aggregation. Möglicherweise bilden auch sie G-Quartett Strukturen durch Assoziation zweier oder mehrerer Oligonucleotide. Solche Strukturen würden gegen Exonuclease Abbau schützen und zu einer erhöhten Aufnahme in die Zelle führen. Da die assoziierten Strukturen immer auch im Gleichgewicht mit den "freien" Oligonucleotiden stehen, stünde auch immer genügend "freies" Oligonucleotid zur Translations- bzw. Transkriptionskontrolle zur Verfügung.

Gegenstand der Erfindung sind Oligonucleotide der Formel

5'-(CAP)-(Oligo)-(CAP)-3'

wobei Oligo für eine Nucleotidsequenz der Länge 10 bis 40 Nucleotide steht und wobei die Nucleotidsequenz des "(Oligo)"-Anteils aus solchen Bereichen von Genen stammt, die für die Transkription der DNA oder die Translation der korrespondierenden mRNA bedeutsam sind. (insbesondere Kann "Oligo" für folgende Sequenzen stehen: CAP für Gₘ, wobei m eine ganze Zahl von null bis zehn, bevorzugt von zwei bis sechs, besonders bevorzugt von drei bis fünf und ganz besonders bevorzugt vier bedeutet und wobei beide im Molekül vorkommenden CAP's unabhängig voneinander definiert sein können und im Falle, wenn m am 5'- oder 3'-Ende gleich null ist und das Ende der "Oligo"-Sequenz von keinem Guanin gebildet wird, verschieden sein müssen.

In den Fällen, in denen das Oligonucleotid (Oligo) am 5'- bzw. 3'-Ende mit einem oder mehreren Guaninen endet, kann es vorteilhaft sein, wenn CAP für G₍ₘ₋ₙ₎ steht, wobei m wie oben definiert ist und n die Zahl der natürlicherweise am 5'- oder 3'-Ende des Oligonucleotids (Oligo) vorkommenden Guanine bedeutet und wobei (m-n) bevorzugt für zwei bis sechs, besonders bevorzugt für drei bis fünf, ganz besonders bevorzugt für vier steht.

Die so abgewandelten Oligonucleotide können unmodifiziert oder modifiziert sein, wobei folgende Varianten zugelassen sein sollen:
a) Vollständiger oder teilweiser Ersatz der 3'- und/oder der 5'-Phosphorsäurediesterbrücken, beispielsweise durch eine Phosphorothioat-, Phosphorodithioat-, (NR¹R²)-Phosphoramidat-, Boranophosphat-, Phosphat-(C₁-C₂₁)-O-Alkylester, Phosphat-[(C₆-C₁₂)Aryl-(C₁-C₂₁)-O-Alkyl]ester, 2,2,2-Trichlorodimethylethylphosphonat-, (C₁-C₈)Alkylphosphonat-, (C₆-C₁₂)-Arylphosphonat-Brücke.
   Bevorzugt ist der Ersatz durch eine Phosphorothioat-, Phosphorodithioat-, NR¹R²-Phosphoramidat-, Phosphat-O-Methylester-, Phosphat-O-ethylester-, Phosphat-O-isopropylester-, Methylphosphonat-, Phenylphosphonat-Brücke. Besonders bevorzugt ist der Ersatz durch eine Phosphorothioat-, Phosphorodithioat-, Methylphosphonat-Brücke.
   Ganz besonders bevorzugt ist der Ersatz durch eine Phosphorothioat-Brücke.
   R¹ und R² stehen unabhängig voneinander für Wasserstoff oder für (C₁-C₁₈)-Alkyl, (C₆-C₂₀)-Aryl, (C₆-C₁₄)-Aryl-(C₁-C₈)-alkyl, -(CH₂)_{c}-[NH(CH₂)_{c}]_{d}-NR³R³ steht, worin c eine ganze Zahl von 2 bis 6 und d eine ganze Zahl von 0 bis 6 ist, und R³ unabhängig voneinander Wasserstoff, (C₁-C₆)-Alkyl oder (C₁-C₄)-Alkoxy-(C₁-C₆)-alkyl ist; bevorzugt steht R¹ und R² für Wasserstoff, (C₁-C₈)-Alkyl oder Methoxyethyl, besonders bevorzugt für Wasserstoff, (C₁-C₄)-Alkyl oder Methoxyethyl. R¹ und R² können auch zusammen mit dem sie tragenden Stickstoffatom einen 5-6-gliedrigen heterocyclischen Ring bilden, der zusätzlich ein weiteres Heteroatom aus der Reihe 0, S, N enthalten kann.
   Vorzugsweise sollen ein, zwei oder drei Phosphorsäurediesterbrücken am 5'-Ende und/oder am 3'-Ende ersetzt werden, bevorzugt am 5'- und am 3'-Ende. Bevorzugt soll auch der Ersatz der Phosphorsäurediesterbrücken an den Pyrimidin-Positionen erfolgen.
b) Vollständiger oder teilweiser Ersatz der 3'- oder 5'-Phosphorsäurediesterbrücken durch "Dephospho"-Brücken [s. z.B. Uhlmann und Peyman in "Methods in Molecular Biology", Vol. 20: "Protocols for Oligonucleotides and Analogs", S. Agrawal, Ed., Humana Press, Totowa 1993, Chapter 16, 355ff], beispielsweise durch Formacetal, 3'-Thioformacetal, Methylhydroxylamin, Oxim, Methylendimethylhydrazo, Dimethylensulfon, Silylgruppen.
   Bevorzugt ist der Ersatz durch Formacetale und 3'-Thioformacetale.
   Vorzugsweise sollen ein, zwei oder drei Phosphorsäurediesterbrücken am 5'-Ende und/oder am 3'-Ende ersetzt werden, bevorzugt am 5'- und am 3'-Ende. Bevorzugt soll auch der Ersatz der Phosphorsäurediesterbrücken an den Pyrimidin-Positionen erfolgen.
c) Vollständiger oder teilweiser Ersatz des Zuckerphosphat-Rückgrats, beispielsweise durch "Morpholinonucleosid"-Oligomere [E. P. Stirchak et al., Nucleic Acids Res. 17: 6129, 1989] oder "Peptide Nucleic Acids" (PNA's) [P. E. Nielsen et al., Bioconj. Chem. 5: 3, 1994], oder auch PNA/DNA-Hydride, wie in der deutschen Patentanmeldung P 44 08 528.1 beschrieben.
d) Vollständiger oder teilweiser Ersatz der β-D-2'-Desoxyriboseeinheiten, beispielsweise durch α-D-2'-Desoxyribose, L-2'-Desoxyribose, 2'-F-2'-Desoxyribose, 2'-O-(C₁-C₆)Alkyl-Ribose, 2'-O-(C₂-C₆)Alkenyl-Ribose, 2'-NH₂-2'-desoxyribose, β-D-Xylofuranose, α-Arabinofuranose, 2,4-Dideoxy-β-D-erythro-hexo-pyranose, und carbocyclische [z.B. Froehler, J.Am.Chem.Soc. 114: 8320, 1992] und offenkettige Zuckeranaloga [z.B. Vandendriessche et al., Tetrahedron 49: 7223, 1993], Bicyclo-Zuckeranaloga [z.B. M. Tarkov et al., Helv. Chim. Acta 76: 481, 1993].
   Bevorzugt ist der Ersatz durch 2'-F-2'-Desoxyribose, 2'-O-(C₁-C₆)Alkyl-Ribose, 2'-O-(C₂-C₆)Alkenyl-Ribose, 2'-NH₂-2'-desoxyribose.
   Besonders bevorzugt ist der Ersatz durch 2'-F-2'-Desoxyribose, 2'-O-(C₁-C₄)Alkyl-Ribose, 2'-O-(C₂-C₄)Alkenyl-Ribose, 2'-NH₂-2'-desoxyribose. Ganz besonders bevorzugt ist der Ersatz durch 2'-O-Methyl-, 2'-O-Allyl-, 2'-O-Butylribose.
   Vorzugsweise sollen ein, zwei oder drei Riboseeinheiten am 5'-Ende und/oder am 3'-Ende ersetzt werden, bevorzugt am 5'- und am 3'-Ende. Bevorzugt soll auch der Ersatz der Riboseeinheiten an den Pyrimidin-Positionen erfolgen.
e) Vollständiger oder teilweiser Ersatz der natürlichen Nucleosid-Basen, beispielsweise durch 5-(Hydroxymethyl)uracil, 5-Aminouracil, Pseudouracil, Dihydrouracil, 5-(C₁-C₆)-Alkyl-uracil, 5-(C₂-C₆)-Alkenyl-uracil, 5-(C₂-C₆)-Alkinyluracil, 5-(C₁-C₆)-Alkyl-cytosin, 5-(C₂-C₆)-Alkenyl-cytosin, 5-(C₂-C₆)-Alkinylcytosin, 5-Fluoruracil, 5-Fluorcytosin, 5-Chloruracil, 5-Chlorcytosin, 5-Bromuracil, 5-Bromcytosin, 7-Deaza-7-substituierte Purine.
   Bevorzugt ist der Ersatz durch 5-(C₁-C₆)-Alkyl-uracil, 5-(C₂-C₆)-Alkenyl-uracil,
   5-(C₂-C₆)-Alkinyl-uracil, 5-(C₁-C₆)-Alkyl-cytosin, 5-(C₂-C₆)-Alkenyl-cytosin,
   5-(C₂-C₆)-Alkinyl-cytosin, 5-Fluoruracil, 5-Fluorcytosin, 5-Chloruracil,
   5-Chlorcytosin, 5-Bromuracil, 5-Bromcytosin, 7-Deaza-7-Alkinyl, bevorzugt hexinyl-substituierte Purine, 7-Deaza-7-Methyl-substituierte Purine, 7-Deaza-7-brom subsitituierte Purine.
   Besonders bevorzugt ist der Ersatz durch 5-(C₃-C₆)-Alkyl-uracil, 5-(C₂-C₆)-Alkenyl-uracil, 5-(C₂-C₆)-Alkinyl-uracil, 5-(C₁-C₆)-Alkyl-cytosin, 5-(C₂-C₆)-Alkenyl-cytosin, 5-(C₂-C₆)-Alkinyl-cytosin. Ganz besonders bevorzugt ist der Ersatz durch 5-Hexinylcytosin, 5-Hexinyluracil, 5-Hexinylcytosin, 5-Propinyluracil, 5-Propinylcytosin. Der Ersatz der Nucleosid-Basen soll nicht in den CAP-Bereichen erfolgen.

Von den obengenannten Modifikationen sind vor allem die Modifikationen aus den Gruppen a), b), c) und d), vor allem aus den Gruppen a) und d), insbesondere aus der Gruppe a) bevorzugt.

Zusätzlich können die erfindungsgemäßen Oligonucleotide beispielsweise am 3'- oder 5'-Ende mit Molekülen verbunden (konjugiert) sein, welche die Eigenschaften von Antisense-Oligonucleotiden oder von Tripelhelix bildenden Oligonucleotiden (wie beispielsweise Zellpenetration, Nucleaseabbau, Affinität zur Target-RNA/DNA, Pharmakokinetik) günstig beeinflußen. Beispiele sind Konjugate mit Poly-Lysin, mit Interkalatoren wie Pyren, Acridin, Phenazin, Phenanthridin, mit fluoreszierenden Verbindungen wie Fluorescein, mit Cross-Linkern wie Psoralen, Azidoproflavin, mit lipophilen Molekülen wie (C₁₂-C₂₀)-Alkyl, mit Lipiden wie 1,2-di-hexadecyl-rac-glycerin, mit Steroiden wie Cholesterin oder Testosteron, mit Vitaminen wie Vitamin E, mit Poly- bzw. Oligo-ethylengylcol, mit (C₁₂-C₁₈)-Alkyl-Phosphatdiestern, mit -O-CH₂-CH(OH)-O-(C₁₂-C₁₈)-Alkyl. Bevorzugt sind Konjugate mit lipophilen Molekülen wie (C₁₂-C₂₀)-Alkyl, mit Steroiden wie Cholesterin oder Testosteron, mit Poly- oder Oligoethylenglykol, mit Vitamin E, mit Interkalatoren wie Pyren, mit (C₁₄-C₁₈)-Alkyl-Phosphatdiestern, mit -O-CH₂-CH(OH)-O-(C₁₂-C₁₆)-Alkyl. Die Darstellung solcher Oligonucleotid-Konjugate ist dem Fachmann bekannt (s. z.B. Uhlmann & Peyman, Chem. Rev. 90: 543, 1990; M. Manoharan in "Antisense Research and Applications", Crooke and Lebleu, Eds., CRC Press, Boca Raton, 1993, Chapter 17, S.303ff, EP 0552766A2).

Weiterhin können die erfindungsgemäßen Oligonucleotide am 3' und/oder am 5'-Ende 3'-3'- und 5'-5'-Inversionen [beschrieben beispielsweise in M. Koga et al., J. Org. Chem. 56: 3757, 1991] tragen.

Gegenstand der Erfindung sind weiterhin Verfahren zur Herstellung der erfindungsgemäßen Verbindungen nach dem Fachmann bekannten Verfahren, insbesondere die chemische Synthese, die Verwendung der erfindungsgemäßen Verbindungen zur Herstellung eines Arzneimittels sowie ein Verfahren zur Herstellung eines Arzneimittels, das dadurch gekennzeichnet ist, daß man die erfindungsgemäßen Oligonucleotide mit einem physiologisch annehmbaren Träger sowie gegebenenfalls geeigneten Zusatz- und/oder Hilfsstoffen vermischt.

Ganz generell erstreckt sich die vorliegende Erfindung auch auf die Verwendung von therapeutisch wirksamen Oligonucleotiden zur Herstellung eines Arzneimittels, bei denen mindestens ein nichtendständiges Pyrimidin-Nucleosid modifiziert ist. Als therapeutisch wirksame Oligonucleotide versteht man im allgemeinen Antisense Oligonucleotide oder Tripelhelix-bildende-Oligonucleotide, insbesondere Antisense-Oligonucleotide.

Darüberhinaus ist ein weiterer Gegenstand der vorliegenden Erfindung die Verwendung von Oligonucleotiden mit mindestens einem nichtendständigen und modifizierten Pyrimidin-Nucleosid als Diagnostikum, beispielsweise zur Detektion der Präsenz oder Absenz oder der Menge eines spezifischen doppelsträngigen oder einzelsträngigen Nucleinsäuremoleküls in einer biologischen Probe.

Die Oligonucleotide haben für die erfindungsgemäße Verwendung eine Länge von 10 bis 40, insbesondere von ca. 12 bis 31 Nucleotiden. Ansonsten gelten auch hier die oben beschriebenen Vorzugsbereiche, Modifikationen bzw. Konjugationen.

Die Arzneimittel der vorliegenden Erfindung können beispielsweise zur Behandlung von Erkrankungen, die durch Viren hervorgerufen werden, beispielsweise durch HIV, HSV-1, HSV-2, Influenza, VSV, Hepatitis B oder Papilloma Viren, verwendet werden.

Erfindungsgemäße Antisense Oligonucleotide, die gegen solche Targets wirksam sind, sind beispielsweise:
a) gegen HIV, z.B.
b) gegen HSV-1, z.B.

Die hier angegebenen arabischen Zahlen beziehen sich auf die weiter vorne angegebenen römischen Zahlen; die hier angegebenen Oligonucleotide sind zusätzlich mit den erfindungsgemäßen CAP's versehen.

Die durch eine Phosphorothioatbrücke (P=S) ersetzten Phosphordiesterbindungen wurden in den Sequenzen mit einem Sternchen (*) markiert.

Die Arzneimittel der vorliegenden Erfindung eignen sich beispielsweise auch zur Behandlung von Krebs oder der Restenose. Beispielsweise können dabei Oligonucleotid-Sequenzen zum Einsatz kommen, die gegen Targets gerichtet sind, die für Krebsentstehung bzw. Krebswachstum verantwortlich sind. Solche Targets sind beispielsweise:
1) Nucleare Onkoproteine wie beispielsweise c-myc, N-myc, c-myb, c-fos, c-fos/jun, PCNA, p120
2) Cytoplasmische/Membran-assoziierte Onkoproteine wie beispielsweise EJ-ras, c-Ha-ras, N-ras, rrg, bcl-2, cdc-2, c-raf-1, c-mos, c-src, c-abl
3) Zelluläre Rezeptoren wie beispielsweise EGF-Rezeptor, c-erbA, Retinoid-Rezeptoren, Protein-Kinase regulatorische Untereinheit, c-fms
4)Cytokine, Wachstumsfaktoren, Extrazelluläre Matrix wie beispielsweise CSF-1, IL-6, IL-1a, IL-1b, IL-2, IL-4, bFGF, Myeloblastin, Fibronectin, VEGF (Vascular Endothelial Growth Factor).

Erfindungsgemäße Antisense-Oligonucleotide, die gegen solche Targets wirksam sind, sind beispielsweise
a) gegen c-Ha-ras, z.B.
b) c-myc, z.B.
c) c-myb, z.B.
d) c-fos, z.B.
e) p120, z.B.
f) EGF-Rezeptor, z.B.
g) p53 Tumorsuppressor, z.B.
h) bFGF, z.B.
i) VEGF, z.B.

Die Arzneimittel der vorliegenden Erfindung eignen sich beispielsweise ferner zu Behandlung von Erkrankungen, die durch Integrine oder Zell-Zell-Adhäsionsrezeptoren beeinflußt werden, beispielsweise durch VLA-4, VLA-2, ICAM oder ELAM.

Erfindungsgemäße Antisense-Oligonucleotide, die gegen solche Targets wirksan sind, sind beispielsweise
a) VLA-4, z.B.
b) ICAM, z.B.
c) ELAM-1, z.B.

Die Arzneimittel der vorliegenden Erfindung eignen sich beispielsweise ferner zur Behandlung von Erkrankungen, die durch Faktoren wie TNF alpha ausgelöst werden.

Erfindungsgemäße Antisense-Oligonucleotide, die gegen solche Targets wirksam sind, sind beispielsweise
a) TNF-alpha, z.B.

Bezüglich der Numerierung der beispielhaften Oligonucleotide und dem Sternchen-Symbol gilt obengesagtes. Die erfindungsgemäßen Oligonucleotide können auch zur Herstellung eines Diagnostikums wenigstens für alle genannten Krankheiten verwendet werden.

Die Arzneimittel können z.B. in Form von pharmazeutischen Präparaten, die man oral, z.B. in Form von Tabletten, Dragees, Hart- oder Weichgelatinekapseln, Lösungen, Emulsionen oder Suspensionen verabreichen kann, verwendet werden. Sie können auch rektal z.B. in Form von Suppositorien oder parenteral z.B. in Form von Injektionslösungen verabreicht werden. Für die Herstellung von pharmazeutischen Präparaten können diese Verbindungen in therapeutisch inerten organischen und anorganischen Trägern verarbeitet werden. Beispiele von solchen Trägern für Tabletten, Dragees und Hartgelatinekapseln sind Laktose, Maisstärke oder Derivate davon, Talg und Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole. Die pharmazeutischen Präparate können auch Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten.
Bevorzugt sind orale Verabreichung und Injektionen. Für die Injektion werden die Antisense-Oligonucleotide in einer flüssigen Lösung, vorzugsweise in einem physiologisch annehmbaren Puffer, wie z.B. Hank's Lösung oder Ringer's Lösung, formuliert. Die Antisense-Oligonucleotide können aber auch in fester Form formuliert werden und vor dem Gebrauch gelöst oder suspendiert werden.

Die für die systematische Verabreichung bevorzugten Dosierungen betragen ca. 0,01 mg/kg bis ca. 50 mg/kg Körpergewicht und Tag.

Die folgenden Beispiele sollen nun die Erfindung näher erläutern. Tabelle 1 zeigt Oligonucleotide, die auf ihre in vitro Aktivität gegen HSV-1 geprüft wurden. Das Oligonucleotid No. 4 ist wie bei Mann et al. (Bioconj. Chem. 3: 554, 1992) beschrieben durch die Einführung eines [4-(1-pyrenyl)butanyl]phosphodiesters am 5'-Ende modifiziert. Die erfindungsgemäßen Oligonucleotide wirken bereits bei einer minimalen Hemmkonzentration von 9 *µ*M (Bspe. 1 und 4) oder sogar schon von 3 *µ*M (Bspe. 2 und 3).

### Beispiel 1

### Oligonucleotidsynthese

Unmodifizierte Oligonucleotide wurden auf einem automatischen DNA Synthesizer (Applied Biosystems Model 380B oder 394) unter Anwendung der Standard Phosphoramidit-Chemie und Oxidation mit Jod synthetisiert. Zur Einführung von Phosphorthioat-Brücken in gemischten Phosphorothioaten und Phosphodiester Oligonucleotiden wurde anstelle von Jod mit TETD (Tetraethylthiuramdisulfid) oxidiert (Applied Biosystems User Bulletin 65). Nach Abspaltung vom festen Träger (CPG oder Tentagel) und Entfernung der Schutzgruppen mit konz. NH₃ bei 55°C während 18h, wurden die Oligonucleotide zunächst durch Butanol-Fällung (Sawadogo, Van Dyke, Nucl. Acids Res. 19: 674, 1991 gereinigt. Anschließend wurde das Natriumsalz erhalten durch Ausfällung aus einer 0.5 M NaCl Lösung mit 2.5 Volumenteilen Ethanol.

Die Analyse der Oligonucleotide erfolgte durch
a) Analytische Gelelektrophorese in 20% Acrylamid, 8M Harnstoff, 45 mM Trisborat Puffer, pH 7.0 und/oder
b) HPLC-Analyse: Waters GenPak FAX, Gradient CH₃CN (400ml), H₂O (1.6l), NaH₂PO₄ (3.1g), NaCl (11.7g), pH6.8 (0.1M an NaCl) nach CH₃CN (400ml), H₂O (1.6l), NaH₂PO₄ (3.1g), NaCl (175.3g), pH6.8 (1.5M an NaCl) und/oder
c) Kapillargelelektrophorese Beckmann Kapillare eCAP™, U100P Gel Column, 65 cm length, 100 mm I.D., window 15 cm from one end, Puffer 140 *µ*M Tris, 360mM Borsäure, 7M Harnstoff und/oder
d) Elektrospray Massenspektroskopie

Die Analyse der Oligonucleotide ergab, daß diese jeweils in einer Reinheit von größer als 90% vorlagen.

### Beispiel 2

### Untersuchung der antiviralen Aktivität von Prüfsubstanzen gegen Herpesviren in vitro

Die antivirale Aktivität der Prüfsubstanzen gegen verschiedene humanpathogene Herpesviren wird im Zellkulturtestsystem untersucht.

Für den Versuch werden Affennierenzellen (Vero, 2x10⁵/ml) in serumhaltigem Dulbecco's MEM (5 % Fötales Kälberserum (FCS)) in 96-Napf-Mikrotiterplatten ausgesät und 24 h bei 37°C und 5 % CO₂ inkubiert. Das serumhaltige Medium wird dann abgesaugt und die Zellen werden zweimal mit serumfreiem Dulbecco's MEM überspült.

Die Testsubstanzen werden in H₂O auf eine Konzentration von 600 *µ*M vorverdünnt und bei -18°C aufbewahrt. Für den Test erfolgen weitere Verdünnungsschritte in Dulbecco's Minimal Essential Medium (MEM). Je 100 *µ*l der einzelnen Prüfsubstanzverdünnungen werden zusammen mit 100 *µ*l serumfreiem Dulbecco's MEM (-FCS) zu den gespülten Zellen gegeben.

Nach 3 h Inkubation bei 37°C und 5 % CO₂ werden die Zellen mit Herpes Simplex Virus Typ 1 (ATCC VR733, HSV-1 F-strain) oder mit Herpes Simplex Virus Typ 2 (ATCC VR734, HSV-2 G-Strain) infiziert in Konzentrationen, bei denen der Zellrasen innerhalb von 3 Tagen vollkommen zerstört wird. Bei HSV-1 beträgt die Infektionsstärke 500 Plaque-bildende Einheiten (PFU) pro Napf, bei HSV-2 350 PFU/Napf. Die Versuchsansätze enthalten dann Prüfsubstanz in Konzentrationen von 80 *µ* M bis 0,04 *µ*M in MEM, ergänzt durch 100 U/ml Penicillin G und 100 mg/l Streptomycin. Alle Versuche werden als Doppelbestimmung durchgeführt mit Ausnahme der Kontrollen, die achtfach je Platte durchgeführt werden.

Die Versuchsansätze werden 17 h bei 37°C und 5 % CO₂ inkubiert. Die Cytotoxizität der Prüfsubstanzen wird nach 20 h Gesamtinkubationszeit durch mikroskopische Begutachtung der Zellkulturen bestimmt. Als Dosis tolerata maxima (DTM) wird die höchste Präparatkonzentration bezeichnet, die unter den genannten Versuchsbedingungen noch keine mikroskopisch erkennbaren Zellschädigungen hervorruft.

Es erfolgt daraufhin die Zugabe von FCS auf eine Endkonzentration von 4 % mit weiterer Inkubation für 55 h bei 37°C und 5 % CO₂. Die unbehandelten Infektionskontrollen zeigen dann einen kompletten cytopathischen Effekt (CPE). Nach mikroskopischer Begutachtung der Zellkulturen werden diese dann mit Neutralrot entsprechend dem Vitalfärbungsverfahren nach Finter (1966) angefärbt. Die antivirale Aktivität einer Testsubstanz wird definiert als minimale Hemmkonzentration (MHK), die benötigt wird, um 30-60 % der Zellen vor dem virusbedingten cytopathogenen Effekt zu schützen.

Tabelle 1: Aktivität von verschieden modifizierten Antisense-Oligonucleotiden gegen HSV-1 in Zellkultur. Die durch eine Phosphorothioatbrücke (P=S) ersetzte Phosphodiesterbindungen wurden in der Sequenz mit ∗ markiert; HMK = minimale Hemmkonzentration; DTM = Dosis tolerata maxima; Py = Pyren.

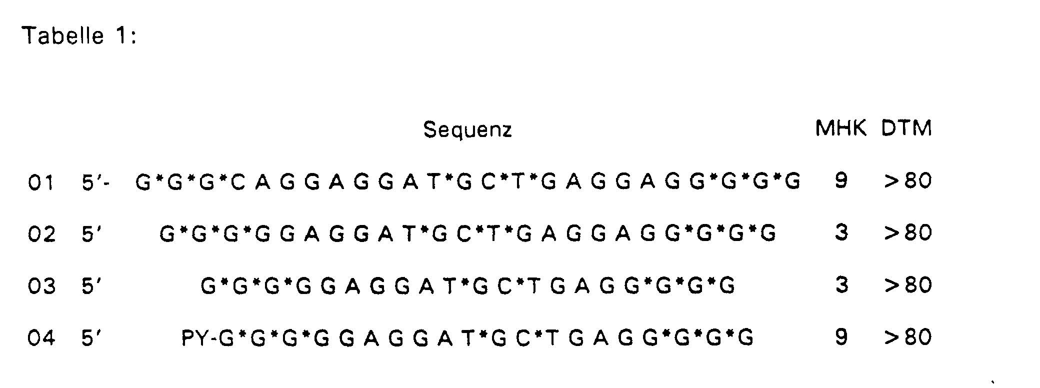

## Patentansprüche

1. Oligonucleotid der Formel
5'-(CAP)-(Oligo)-(CAP)-3'
wobei (Oligo) für eine Nucleotidsequenz der Länge 10 bis 40 Nucleotide steht und wobei die Nucleotidsequenz des "(Oligo)"-Anteils aus solchen Bereichen von Genen stammt, die für die Transkription der DNA oder die Translation der korrespondierenden mRNA bedeutsam sind; und CAP für Gₘ steht,
wobei m für eine ganze Zahl von null bis zehn steht und
wobei beide CAP's unabhängig voneinander definiert sein können und, falls m am 5'-oder 3'-Ende null ist, die CAP's verschieden sein müssen und
wobei das Ende der "Oligo"-Sequenz von keinem Guanin gebildet wird.

2. Oligonukleotid nach Anspruch 1, wobei m für eine ganze Zahl von zwei bis sechs steht.

3. Oligonukleotid nach Anspruch 1, wobei m für drei bis fünf steht.

4. Oligonukleotid nach Anspruch 1, wobei m für vier steht.

5. Oligonukleotid der Formel
5'-(CAP)-(Oligo)-(CAP)-3'
wobei (Oligo) für eine Nukleotidsequenz der Länge 10 bis 40 Nukleotide steht und wobei die Nukleotidsequenz des "(Oligo)"-Anteils aus solchen Bereichen von Genen stammt, die für die Transkription der DNA oder die Translation der korrespondierenden mRNA bedeutsam sind und wobei das (Oligo) am 5'- bzw. 3'-Ende mit einem oder mehreren Guaninen endet;
CAP für G₍ₘ₋ₙ₎ steht,
wobei m wie in Anspruch 1 definiert ist und n die Zahl der natürlicherweise am 5'- oder 3'-Ende des Oligonukleotids (Oligo) vorkommenden Guanine bedeutet und wobei (m-n) für zwei bis sechs steht und
wobei beide im Molekül vorkommenden CAP's unabhängig voneinander definiert sein können.

6. Oligonukleotid nach Anspruch 5, wobei (m-n) für drei bis fünf steht.

7. Oligonukleotid nach Anspruch 5, wobei (m-n) für vier steht.

8. Oligonukleotid nach einem oder mehreren der Ansprüche 1 bis 7, wobei das Oligonukleotid modifiziert ist.

9. Oligonukleotid nach einem oder mehreren der Ansprüche 1 bis 8, wobei 2 bis 10 nichtendständige Pyrimidin-Nukleoside modifiziert sind.

10. Oligonukleotid nach einem oder mehreren der Ansprüche 1 bis 9, wobei das Oligonukleotid am 5'-endständigen und/oder am 3'-endständigen Nukleotid modifiziert ist.

11. Oligonukleotid nach einem oder mehreren der Ansprüche 1 bis 10, wobei ein oder mehrere Gruppen von mindestens 1 bis 4 miteinander verbundenen Nukleotiden nicht modifiziert sind.

12. Oligonukleotid nach einem oder mehreren der Ansprüche 1 bis 11, wobei die Modifikation definiert ist als
(a) vollständiger oder teilweiser Ersatz der 3'- und/oder 5'-Phosphorsäurediesterbrücken und/oder
(b) vollständiger oder teilweiser Ersatz der 3'- oder 5'-Phosphorsäurediesterbrücken durch "Dephospho"-Brücken und/oder
(c) vollständiger oder teilweiser Ersatz des Zuckerphosphat-Rückgrats und/oder
(d) vollständiger oder teilweiser Ersatz der β-D-2'-Desoxyriboseeinheiten und/oder
(e) vollständiger oder teilweiser Ersatz der natürlichen Nucleosid-Basen.

13. Oligonucleotid nach einem oder mehreren der Ansprüche 1 bis 12, wobei die Modifikation definiert ist als
(a) vollständiger oder teilweiser Ersatz der 3'- und/oder 5'-Phosphorsäurediesterbrücken durch Phosphorothioat-, Phosphorodithioat-, (NR¹R²)-Phosphoramidat-, Boranophosphat-, Phosphat-(C₁-C₂₁)-O-Alkylester, Phosphat-[(C₆-C₁₂)Aryl-(C₁-C₂₁)-O-Alkyl]ester-, 2,2,2-Trichlorodimethylethylphosphonat-, (C₁-C₈)Alkylphosphonat-, (C₆-C₁₂)-Arylphosphonat-Brücken,
wobei
R¹ und R² unabhängig voneinander für Wasserstoff oder für (C₁-C₁₈)-Alkyl, (C₆-C₂₀)-Aryl, (C₆-C₁₄)-Aryl-(C₁-C₈)-alkyl, -(CH₂)_{c}-[NH(CH₂)_{c}]_{d}-NR³R³ steht, worin c eine ganze Zahl von 2 bis 6 und d eine ganze Zahl von 0 bis 6 ist, und R³ unabhängig voneinander Wasserstoff, (C₁-C₆)-Alkyl oder (C₁-C₄)-Alkoxy-C₁-C₆-alkyl ist oder R¹ und R² zusammen mit dem sie tragenden Stickstoffatom einen 5-6-gliedrigen heterocyclischen Ring bilden, der zusätzlich ein weiteres Heteroatom aus der Reihe O, S, N enthalten kann; und/oder
(b) vollständiger oder teilweiser Ersatz der 3'- und/oder 5'-Phosphorsäurediesterbrücken durch Formacetal, 3'-Thioformacetal, Methylhydroxylamin, Oxim, Methylendimethylhydrazo, Dimethylensulfon oder Silylgruppen und/oder
(c) vollständiger oder teilweiser Ersatz des Zuckerphosphat-Rückgrats durch "Morpholinonucleosid"-Oligomere, PNA's oder PNA-DNA-Hybride und/oder
(d) vollständiger oder teilweiser Ersatz der β-D-2'-Deoxyriboseeinheiten durch α-D-2'-Desoxyribose, L-2'-Desoxyribose, 2'-F-2'-Desoxyribose, 2'-O- (C₁-C₆)Alkyl-Ribose, 2'-O-(C₂-C₆)Alkenyl-Ribose, 2'-NH₂-2'-desoxyribose, β-D-Xylofuranose, α-Arabinofuranose, 2,4-Dideoxy-β-D-erythro-hexo-pyranose, carbocyclische, offenkettige oder Bicyclo-Zuckeranaloga, und/oder
(e) vollständiger oder teilweise Ersatz der natürlichen Nukleosid-Basen durch 5-(Hydroxymethyl)uracil, 5-Aminouracil, Pseudouracil, Dihydrouracil, 5-(C₁-C₆)-Alkyluracil, 5-(C₂-C₆)-Alkenyl-uracil, 5-(C₂-C₆)-Alkinyl-uracil, 5-(C₁-C₆)-Alkyl-cytosin, 5-(C₂-C₆)-Alkenyl-cytosin, 5-(C₂-C₆)-Alkinyl-cytosin, 5-Fluoruracil, 5-Fluorcytosin, 5-Chloruracil, 5-Chlorcytosin, 5-Bromuracil, 5- Bromcytosin,
wobei der Ersatz der Nucleosid-Basen nicht in den CAP-Bereichen erfolgen soll.

14. Oligonukleotid nach einem oder mehreren der Ansprüche 1 bis 13, wobei die Modifikation definiert ist als
(a) der teilweise oder vollständige Ersatz der 3'- und/oder 5'-Phosphorsäurediesterbrücken durch Phosphat-O-Methylester, Phosphat-O-ethylester-, Phosphat-O-isopropylester-, Methylphosphonat- und/oder Phenylphosphonat-Brücken und/oder
(b) der teilweise oder vollständige Ersatz der β-D-2'-Deoxyriboseeinheiten durch 2'-O-Methyl-, 2'-O-Allyl- und/oder 2'-O-Butylribose und/oder
(c) der teilweise oder vollständige Ersatz der natürlichen Nukleosid-Basen durch 5-Pentinylcytosin, 5-Hexinyluracil und/oder 5-Hexinylcytosin, wobei der Ersatz der Nukleosid-Basen nicht in den CAP-Bereichen erfolgen soll.

15. Oligonukleotid nach einem oder mehreren der Ansprüche 1 bis 14, wobei zwei oder drei Phosphorsäurediesterbrücken am 5'-Ende und/oder am 3'-Ende des Oligonukleotids ersetzt sind.

16. Oligonukleotid nach einem oder mehreren der Ansprüche 1 bis 12, wobei Phosphorsäurediester-Brücken an Pyrimidin-Positionen ersetzt sind.

17. Oligonukleotid nach einem oder mehreren der Ansprüche 15 und 16, wobei die Phosphorsäurediesterbrücken durch Phosphorothioatbrücken ersetzt sind.

18. Oligonukleotid nach einem oder mehreren der Ansprüche 1 bis 17, wobei zwei oder drei Riboseeinheiten am 5'-Ende und/oder am 3'-Ende ersetzt sind.

19. Oligonukleotid nach einem oder mehreren der Ansprüche 1 bis 18, wobei Riboseeinheiten an Pyrimidinpositionen ersetzt sind.

20. Oligonukleotid nach einem oder mehreren der Ansprüche 1 bis 19, wobei das Oligonukleotid am 3'- und/oder 5'-Ende mit Molekülen, welche die Zellpenetration, Nukleaseresistenz, Affinität zur Target-RNA/DNA oder Pharmakinetik günstig beeinflussen, verbunden ist.

21. Oligonukleotid nach einem oder mehreren der Ansprüche 1 bis 20, wobei das Oligonukleotid am 5'- und/oder am 3'-Ende mit Molekülen ausgewählt aus Poly-Lysin, Interkalatoren, fluoreszierenden Verbindungen, Linkern, lipophilen Molekülen, Lipiden, Steroiden, Vitaminen, Poly- bzw. Oligoethylenglykol, (C₁₂-C₁₈)-Alkyl-Phosphatdiestern oder -O-CH₂-CH(OH)-O-(C₁₂-C₁₈)-Alkyl-Resten, verbunden ist.

22. Oligonukleotid nach einem oder mehreren der Ansprüche 1 bis 21, wobei das Oligonukleotid am 5'- und/oder am 3'-Ende mit Molekülen ausgewählt aus Pyren, Acridin, Phenazin, Phenanthridin, Fluorescein, Psoralen, Azidoproflavin, (C₁₂-C₂₀)-Alkyl, 1,2-di-hexadecyl-rac-glycerin, Cholesterin, Testosteron oder Vitamin E verbunden ist.

23. Oligonukleotid nach einem oder mehreren der Ansprüche 1 bis 22, wobei das Oligonukleotid am 3'- und/oder 5'-Ende 3'-3'- bzw. 5'-5'-Inversionen trägt.

24. Oligonucleotide nach einem oder mehreren der Ansprüche 1 bis 23, wobei (Oligo) für eine der Sequenzen steht.

25. Oligonucleotid mit einer der Sequenzen oder wobei "*" für eine Phosphorothioatbrücke und "PY" für Pyren steht.

26. Oligonukleotid nach einem oder mehreren der Ansprüche 1 bis 24, wobei das Oligonukleotid eine Sequenz hat, die gegen ein Target gerichtet ist, das für Krebsentstehung bzw. Krebswachstum verantwortlich ist.

27. Verfahren zur Herstellung eines Oligonukleotids nach einem oder mehreren der Ansprüche 1 bis 26, **dadurch gekennzeichnet, daß** das Oligonukleotid chemisch an einem festen Träger synthetisiert wird.

28. Arzneimittel enthaltend ein oder mehrere Oligonucleotide gemäß einem oder mehreren der Ansprüche 1 bis 26 und einen physiologisch annehmbaren Träger
sowie gegebenenfalls geeignete Zusatz- und/oder Hilfsstoffe.

29. Verwendung eines Oligonucleotids gemäß einem oder mehreren der Ansprüche 1 bis 26 zur Herstellung eines Arzneimittels.

30. Verwendung eines Oligonucleotids gemäß einem oder mehreren der Ansprüche 1 bis 26 zur Herstellung eines Arzneimittels zur Behandlung von Erkrankungen, die durch Viren hervorgerufen werden, zur Behandlung von Krebs, Restenose oder von Erkrankungen, die durch Integrine oder Zell-Zell-Adhäsionsrezeptoren beeinflußt werden oder die durch diffusible Faktoren ausgelöst werden.

31. Verwendung eines Oligonucleotids gemäß einem oder mehreren der Ansprüche 1 bis 26 zur Herstellung eines Diagnostikums.

32. Verwendung eines Oligonucleotids gemäß einem oder mehreren der Ansprüche 1 bis 26 zur Herstellung eines Diagnostikums für Erkrankungen, die durch Viren hervorgerufen werden, für Krebs, Restenose oder für Erkrankungen, die durch Integrine oder Zell-Zell-Adhäsionsrezeptoren beeinflußt werden oder durch diffusible Faktoren ausgelöst werden.

## Claims

1. An oligonucleotide of the formula
5'-(CAP)-(oligo)-(CAP)-3'
wherein (oligo) is a nucleotide sequence of from 10 to 40 nucleotides in length and
wherein the nucleotide sequence of the "(oligo)" moiety is derived from those regions of genes which are of importance for the transcription of the DNA or the translation of the corresponding mRNA;
and CAP is Gₘ,
wherein m is a whole number from zero to ten, and
wherein the two CAPs can be defined independently of each other and, if m is zero at the 5' end or 3' end, the CAPs have to be different, and
wherein the end of the "oligo" sequence is not formed by a guanine.

2. An oligonucleotide as claimed in claim 1, wherein m is a whole number from two to six.

3. An oligonucleotide as claimed in claim 1, wherein m is from three to five.

4. An oligonucleotide as claimed in claim 1, wherein m is four.

5. An oligonucleotide of the formula
5'-(CAP)-(oligo)-(CAP)-3'
wherein (oligo) is a nucleotide sequence of from 10 to 40 nucleotides in length and
wherein the nucleotide sequence of the "(oligo)" moiety is derived from those regions of genes which are of importance for the transcription of the DNA or the translation of the corresponding mRNA
and wherein the (oligo) ends at the 5' end and/or 3' end with one or more guanines;
CAP is G₍ₘ₋ₙ₎,
wherein m is defined as in claim 1 and n is the number of the guanines which are naturally present at the 5' end or 3' end of the oligonucleotide (oligo), and wherein (m-n) is from two to six, and
wherein the two CAPs which are present in the molecule can be defined independently of each other.

6. An oligonucleotide as claimed in claim 5, wherein (m - n) is from three to five.

7. An oligonucleotide as claimed in claim 5, wherein (m - n) is four.

8. An oligonucleotide as claimed in one or more of claims 1 to 7, wherein the oligonucleotide is modified.

9. An oligonucleotide as claimed in one or more of claims 1 to 8, wherein from 2 to 10 non-terminal pyrimidine nucleosides are modified.

10. An oligonucleotide as claimed in one or more of claims 1 to 9, wherein the oligonucleotide is modified at the 5'-terminal and/or the 3'-terminal nucleotide.

11. An oligonucleotide as claimed in one or more of claims 1 to 10, wherein one or more groups which have at least 1 to 4 nucleotides which are linked together are not modified.

12. An oligonucleotide as claimed in one or more of claims 1 to 11, wherein the modification is defined as
(a) complete or partial replacement of the 3'- and/or
5'-phosphoric acid diester bridges and/or
(b) complete or partial replacement of the 3'- or 5'-phosphoric acid diester bridges with "dephospho" bridges and/or
(c) complete or partial replacement of the sugar phosphate backbone and/or
(d) complete or partial replacement of the β-D-2'-deoxyribose units and/or
(e) complete or partial replacement of the natural nucleoside bases.

13. An oligonucleotide as claimed in one or more of claims 1 to 12, wherein the modification is defined as
(a) complete or partial replacement of the 3'- and/or 5'-phosphoric acid diester bridges with phosphorothioate bridges, phosphorodithioate bridges, (NR¹R²)-phosphoramidate bridges, boranophosphate bridges, phosphate-(C₁-C₂₁)-O-alkyl ester bridges, phosphate-[(C₆-C₁₂)aryl-(C₁-C₂₁)-O-alkyl] ester bridges, 2,2,2-trichlorodimethylethylphosphonate bridges, (C₁-C₆)alkylphosphonate bridges or (C₆-C₁₂)arylphosphonate bridges,
wherein
R¹ and R² are, independently of each other, hydrogen or (C₁-C₁₈)-alkyl, (C₆-C₂₀)-aryl, (C₆-C₁₄)-aryl-(C₁-C₈)-alkyl or -(CH₂)_{c}-[NH(CH₂)_{c}]_{d}-NR³R³, in which c is a whole number from 2 to 6 and d is a whole number from 0 to 6, and R³ is, independently of each other, hydrogen, (C₁-C₆)-alkyl or (C₁-C₄)-alkoxy-C₁-C₆-alkyl, or R¹ and R² form, together with the nitrogen atom carrying them, a 5-6-membered heterocyclic ring which can additionally contain a further heteroatom from the series O, S and N; and/or
(b) complete or partial replacement of the 3'- and/or 5'-phosphoric acid diester bridges with formacetal, 3'-thioformacetal, methylhydroxylamine, oxime, methylenedimethylhydrazo, dimethylenesulfone or silyl groups, and/or
(c) complete or partial replacement of the sugar phosphate backbone with "morpholinonucleoside" oligomers, PNAs or PNA-DNA hybrids, and/or
(d) complete or partial replacement of the β-D-2'-deoxyribose units with α-D-2'-deoxyribose, L-2'-deoxyribose, 2'-F-2'-deoxyribose, 2'-O- (C₁-C₆)alkyl-ribose, 2'-O-(C₂-C₆)alkenyl-ribose, 2'-NH₂-2'-deoxyribose, β-D-xylofuranose, α-arabinofuranose, 2,4-dideoxy-β-D-erythrohexopyranose, or carbocyclic, open-chain or bicyclo sugar analogs, and/or
(e) complete or partial replacement of the natural nucleoside bases with 5-(hydroxymethyl)uracil, 5-aminouracil, pseudouracil, dihydrouracil, 5-(C₁-C₆)-alkyl-uracil, 5-(C₂-C₆)-alkenyl-uracil, 5-(C₂-C₆)-alkynyl-uracil, 5-(C₁-C₆)-alkyl-cytosine, 5-(C₂-C₆)-alkenyl-cytosine, 5-(C₂-C₆)-alkynyl-cytosine, 5-fluorouracil, 5-fluorocytosine, 5-chlorouracil, 5-chlorocytosine, 5-bromouracil or 5-bromocytosine,
wherein the nucleoside bases are not to be replaced in the CAP regions.

14. An oligonucleotide as claimed in one or more of claims 1 to 13, wherein the modification is defined as
(a) the partial or complete replacement of the 3'-and/or 5'-phosphoric acid diester bridges with phosphate-O-methyl ester bridges, phosphate-O-ethyl ester bridges, phosphate-O-isopropyl ester bridges, methylphosphonate bridges and/or phenylphosphonate bridges, and/or
(b) the partial or complete replacement of the β-D-2'-deoxyribose units with 2'-O-methyl-, 2'-O-allyl-and/or 2'-O-butylribose, and/or
(c) the partial or complete replacement of the natural nucleoside bases with 5-pentynylcytosine, 5-hexynyluracil and/or 5-hexynylcytosine, wherein the nucleoside bases are not to be replaced in the CAP regions.

15. An oligonucleotide as claimed in one or more of claims 1 to 14, wherein two or three phosphoric acid diester bridges are replaced at the 5' end and/or 3' end of the oligonucleotide.

16. An oligonucleotide as claimed in one or more of claims 1 to 12, wherein phosphoric acid diester bridges are replaced at pyrimidine positions.

17. An oligonucleotide as claimed in one or both of claims 15 and 16, wherein the phosphoric acid diester bridges are replaced with phosphorothioate bridges.

18. An oligonucleotide as claimed in one or more of claims 1 to 17, wherein two or three ribose units are replaced at the 5' end and/or the 3' end.

19. An oligonucleotide as claimed in one or more of claims 1 to 18, wherein ribose units are replaced at pyrimidine positions.

20. An oligonucleotide as claimed in one or more of claims 1 to 19, wherein the oligonucleotide at the 3' end and/or 5' end is linked to molecules which promote cell penetration, nuclease resistance, affinity for the target RNA/DNA or pharmacokinetics.

21. An oligonucleotide as claimed in one or more of claims 1 to 20, wherein the oligonucleotide at the 5' end and/or 3' end is linked to molecules which are selected from polylysine, intercalators, fluorescent compounds, linkers, lipophilic molecules, lipids, steroids, vitamins, polyethylene glycol or oligoethylene glycol, (C₁₂-C₁₈)-alkyl-phosphate diesters or -O-CH₂-CH(OH)-O-(C₁₂-C₁₈)-alkyl radicals.

22. An oligonucleotide as claimed in one or more of claims 1 to 21, wherein the oligonucleotide at the 5' end and/or 3' end is linked to molecules which are selected from pyrene, acridine, phenazine, phenanthridine, fluorescein, psoralen, azidoproflavine, (C₁₂-C₂₀)-alkyl, 1,2-dihexadecyl-rac-glycerol, cholesterol, testosterone or vitamin E.

23. An oligonucleotide as claimed in one or more of claims 1 to 22, wherein the oligonucleotide carries 3'-3' and/or 5'-5' inversions at the 3' end and/or 5' end.

24. An oligonucleotide as claimed in one or more of claims 1 to 23, wherein (oligo) is one of the sequences

25. An oligonucleotide having one of the sequences or wherein "*" is a phosphorothioate bridge and "PY" is pyrene.

26. An oligonucleotide as claimed in one or more of claims 1 to 24, wherein the oligonucleotide has a sequence which is directed against a target which is responsible for the origin of cancer and/or the growth of cancer.

27. A process for preparing an oligonucleotide as claimed in one or more of claims 1 to 26, wherein the oligonucleotide is synthesized chemically on a solid support.

28. A drug which comprises one or more oligonucleotides as claimed in one or more of claims 1 to 26 and a physiologically acceptable excipient and also, where appropriate, suitable additives and/or adjuvants.

29. The use of an oligonucleotide as claimed in one or more of claims 1 to 26 for producing a drug.

30. The use of an oligonucleotide as claimed in one or more of claims 1 to 26 for producing a drug for treating diseases which are caused by viruses and for treating cancer, restenosis or diseases which are influenced by integrins or cell-cell adhesion receptors or which are triggered by diffusible factors.

31. The use of an oligonucleotide as claimed in one or more of claims 1 to 26 for preparing a diagnostic agent.

32. The use of an oligonucleotide as claimed in one or more of claims 1 to 26 for preparing a diagnostic agent for diseases which are caused by viruses, for cancer, for restenosis or for diseases which are influenced by integrins or cell-cell adhesion receptors or triggered by diffusible factors.

## Revendications

1. Oligonucléotide de formule
5'-(CAP)-(Oligo)-(CAP)-3'
dans laquelle (Oligo) représente une séquence de nucléotides de 10 à 40 nucléotides de longueur et la séquence de nucléotides de la fraction "Oligo" provient de domaines de gènes importants pour la transcription de l'ADN ou la traduction de l'ARNm correspondant;
et CAP représente Gₘ, où m est un nombre entier de 0 à 10, et
les deux CAP peuvent être définis indépendamment l'un de l'autre, et, dans le cas où m est 0 à l'extrémité 5' ou 3', les CAP doivent être différents, et
l'extrémité de la séquence de "Oligo" n'est pas formée d'une guanine.

2. Oligonucléotide selon la revendication 1, dans lequel m est un nombre entier de 2 à 6.

3. Oligonucléotide selon la revendication 1, dans lequel m est un nombre de 3 à 5.

4. Oligonucléotide selon la revendication 1, dans lequel m est égal à 4.

5. Oligonucléotide de formule
5'-(CAP)-(Oligo)-(CAP)-3'
dans laquelle (Oligo) représente une séquence de nucléotides de 10 à 40 nucléotides de longueur et la séquence de nucléotides de la fraction "Oligo" provient de domaines de gènes importants pour la transcription de l'ADN ou la traduction de l'ARNm correspondant, la séquence (Oligo) se terminant à l'extrémité 5' ou 3' par une ou plusieurs guanines;
CAP représente G₍ₘ₋ₙ₎,
où m a la définition donnée dans la revendication 1 et n est le nombre de guanines présentes naturellement à l'extrémité 5' ou 3' de l'oligonucléotide (Oligo), et (m-n) est égal à 2 à 6, et
les deux CAP présents dans la molécule peuvent être définis indépendamment l'un de l'autre.

6. Oligonucléotide selon la revendication 5, dans lequel (m-n) est un nombre de 3 à 5.

7. Oligonucléotide selon la revendication 5, dans lequel (m-n) est égal à 4.

8. Oligonucléotide selon l'une ou plusieurs des revendications 1 à 7, l'oligonucléotide étant modifié.

9. Oligonucléotide selon l'une ou plusieurs des revendications 1 à 8, dans lequel 2 à 10 nucléosides pyrimidiques non terminaux sont modifiés.

10. Oligonucléotide selon l'une ou plusieurs des revendications 1 à 9, l'oligonucléotide étant modifié au niveau du nucléotide terminal de l'extrémité 5' et/ou de l'extrémité 3'.

11. Oligonucléotide selon l'une ou plusieurs des revendications 1 à 10, dans lequel un ou plusieurs groupes d'au moins 1 à 4 nucléotides liés entre eux ne sont pas modifiés.

12. Oligonucléotide selon l'une ou plusieurs des revendications 1 à 11, dans lequel la modification est définie comme
(a) le remplacement total ou partiel des ponts phosphodiester en 3' et/ou 5' et/ou
(b) le remplacement total ou partiel des ponts phosphodiester en 3' et/ou 5' par des ponts "déphospho" et/ou
(c) le remplacement total ou partiel du squelette de phosphates de sucres et/ou
(d) le remplacement total ou partiel des unités β-D-2'-désoxyribose et/ou
(e) le remplacement total ou partiel des bases de nucléosides naturelles.

13. Oligonucléotide selon l'une ou plusieurs des revendications 1 à 12, dans lequel la modification est définie comme
(a) le remplacement total ou partiel des ponts phosphodiester en 3' et/ou 5' par des ponts phosphorothioate, phosphorodithioate, (NR¹R²)-phosphoramidate, boranophosphate, phosphate de O-alkyle en C₁-C₂₁, phosphate de [(aryl en C₆-C₂₀)-O-(alkyle en C₁-C₂₁)], phosphonate de 2,2,2-trichlorodiméthyléthyle, phosphonate d'alkyle en C₁-C₈, phosphonate d'aryle en C₆-C₁₂, où
R¹ et R² représentent indépendamment l'un de l'autre un atome d'hydrogène ou un groupe alkyle en C₁-C₁₈, aryle en C₆-C₂₀, (aryl en C₆-C₁₄)-(alkyle en C₁-C₈), -(CH₂)_{c}-[NH(CH₂)_{c}]_{d}-NR³R³, où c est un nombre entier de 2 à 6 et d un nombre entier de 0 à 6, et les R³ représentent, indépendamment l'un de l'autre, un atome d'hydrogène ou un groupe alkyle en C₁-C₆ ou (alcoxy en C₁-C₄)-(alkyle en C₁-C₆), ou R¹ et R² forment ensemble, avec l'atome d'azote qui les porte, un cycle hétérocyclique de 5 à 6 chaînons pouvant contenir en outre un autre hétéroatome de la série O, S, N; et/ou
(b) le remplacement total ou partiel des ponts phosphodiester en 3' et/ou 5' par du formacétal, du 3'-thioformacétal, de la méthylhydroxylamine, une oxime, un groupe méthylènediméthylhydrazo, une diméthylènesulfone ou des groupes silyle et/ou
(c) le remplacement total ou partiel du squelette de phosphates de sucres par des oligomères de "morpholinonucléosides", des PNA ou des hybrides PNA-ADN, et/ou
(d) le remplacement total ou partiel des unités β-D-2'-désoxyribose par des unités α-D-2'-désoxyribose, L-2'-désoxyribose, 2'-F-2'-désoxyribose, 2'-O-(alkyl en C₁-C₆)ribose, 2'-O-(alcényl en C₂-C₆)ribose, 2'-NH₂-2'-désoxyribose, β-D-xylofuranose, α-arabinofuranose, 2,4-didésoxy-β-D-érythro-hexopyranose, des analogues de sucres carbocycliques, linéaires ou bicyclo, et/ou
(e) le remplacement total ou partiel des bases de nucléosides naturelles par du 5-(hydroxyméthyl)uracile, du 5-aminouracile, du pseudouracile, du dihydrouracile, un 5-(alkyl en C₁-C₆)uracile, un 5-(alcényl en C₂-C₆)uracile, un 5-(alcynyl en C₂-C₆)uracile, une 5-(alkyl en C₁-C₆)cytosine, une 5-(alcényl en C₂-C₆)cytosine, une 5-(alcynyl en C₂-C₆)cytosine, du 5-fluorouracile, de la 5-fluorocytosine, du 5-chlorouracile, de la 5-chlorocytosine, du 5-bromouracile, de la 5-bromocytosine, le remplacement des bases de nucléosides ne devant pas se faire dans les domaines CAP.

14. Oligonucléotide selon l'une ou plusieurs des revendications 1 à 13, dans lequel la modification est définie comme
(a) le remplacement total ou partiel des ponts phosphodiester en 3' et/ou 5' par des ponts phosphate de O-méthyle, phosphate de O-éthyle, phosphate de O-isopropyle, méthylphosphonate et/ou phénylphosphonate; et/ou
(b) le remplacement total ou partiel des unités β-D-2'-désoxyribose par du 2'-O-méthylribose, du 2'-O-allylribose et/ou du 2'-O-butylribose et/ou
(c) le remplacement total ou partiel des bases de nucléosides naturelles par la 5-pentynylcytosine, le 5-hexynyluracile et/ou la 5-hexynylcytosine, le remplacement de bases de nucléosides ne devant pas se faire dans les domaines CAP.

15. Oligonucléotide selon l'une ou plusieurs des revendications 1 à 14, dans lequel 2 ou 3 ponts phosphodiester à l'extrémité 5' et/ou à l'extrémité 3' de l'oligonucléotide sont remplacés.

16. Oligonucléotide selon l'une ou plusieurs des revendications 1 à 12, dans lequel des ponts phosphodiester sont remplacés au niveau des positions des pyrimidines.

17. Oligonucléotide selon l'une ou plusieurs des revendications 15 et 16, dans lequel les ponts phosphodiester sont remplacés par des ponts phosphorothioate.

18. Oligonucléotide selon l'une ou plusieurs des revendications 1 à 17, dans lequel deux ou trois unités de ribose sont remplacées à l'extrémité 5' et/ou à l'extrémité 3'.

19. Oligonucléotide selon l'une ou plusieurs des revendications 1 à 18, dans lequel des unités ribose sont remplacées au niveau des positions des pyrimidines.

20. Oligonucléotide selon l'une ou plusieurs des revendications 1 à 19, l'oligonucléotide étant lié à l'extrémité 3' et/ou 5' avec des molécules qui ont un effet favorable sur la pénétration des cellules, la résistance aux nucléases, l'affinité pour l'ARN/ADN cible ou la pharmacocinétique.

21. Oligonucléotide selon l'une ou plusieurs des revendications 1 à 20, l'oligonucléotide étant lié à l'extrémité 5' et/ou 3' avec des molécules choisies parmi une polylysine, des espaceurs, des composés fluorescents, des lieurs, des molécules lipophiles, des lipides, des stéroïdes, des vitamines, du polyéthylèneglycol ou de l'oligoéthylèneglycol, des phosphates de di(alkyle en C₁₂-C₁₈) ou des restes -O-CH₂-CH(OH)-O-(alkyle en C₁₂-C₁₈).

22. Oligonucléotide selon l'une ou plusieurs des revendications 1 à 21, l'oligonucléotide étant lié à l'extrémité 5' et/ou 3' avec des molécules choisies parmi le pyrène, l'acridine, la phénazine, la phénanthridine, la fluorescéine, le psoralène, l'azidoproflavine, un alkyle en C₁₂-C₂₀, le 1,2-dihexadécyl-rac-glycérol, le cholestérol, la testostérone ou la vitamine E.

23. Oligonucléotide selon l'une ou plusieurs des revendications 1 à 22, l'oligonucléotide portant des inversions 3'-3' ou 5'-5' à l'extrémité 3' et/ou 5'.

24. Oligonucléotide selon l'une ou plusieurs des revendications, dans lequel (Oligo) représente l'une des séquences

25. Oligonucléotide ayant l'une des séquences ou dans lesquelles "*" indique un pont phosphorothioate et "PY" représente le pyrène.

26. Oligonucléotide selon l'une ou plusieurs des revendications 1 à 24, l'oligonucléotide ayant une séquence dirigée contre une cible qui est responsable de l'apparition d'un cancer ou du développement d'un cancer.

27. Procédé de préparation d'un oligonucléotide selon l'une ou plusieurs des revendications 1 à 26, **caractérisé en ce que** l'on synthétise l'oligonucléotide par voie chimique sur un support solide.

28. Médicament contenant un ou plusieurs oligonucléotides selon l'une ou plusieurs des revendications 1 à 26 et un support physiologiquement acceptable, ainsi qu'éventuellement des additifs et/ou des agents auxiliaires appropriés.

29. Utilisation d'un oligonucléotide selon l'une ou plusieurs des revendications 1 à 26 pour la préparation d'un médicament.

30. Utilisation d'un oligonucléotide selon l'une ou plusieurs des revendications 1 à 26 pour la préparation d'un médicament destiné au traitement de maladies provoquées par des virus ou au traitement du cancer, de la resténose ou de maladies influencées par des intégrines ou des récepteurs d'adhésion cellule-cellule, ou déclenchées par des facteurs diffusibles.

31. Utilisation d'un oligonucléotide selon l'une ou plusieurs des revendications 1 à 26 pour la préparation d'un agent de diagnostic.

32. Utilisation d'un oligonucléotide selon l'une ou plusieurs des revendications 1 à 26 pour la préparation d'un agent de diagnostic pour des maladies provoquées par des virus, pour le cancer, la resténose ou pour des maladies influencées par des intégrines ou des récepteurs d'adhésion cellule-cellule, ou déclenchées par des facteurs diffusibles.


### SEQUENZPROTOKOLL

(1) ALLGEMEINE INFORMATION:
   (i) ANMELDER:
      (A) NAME: Hoechst Aktiengesellschaft
      (B) STRASSE: -
      (C) ORT: Frankfurt
      (D) BUNDESLAND: -
      (E) LAND: Deutschland
      (F) POSTLEITZAHL: 65926
      (G) TELEPHON: 069-305-3005
      (H) TELEFAX: 069-357175
      (I) TELEX: -
   (ii) ANMELDETITEL: G-Cap Stabilisierte Oligonucleotide
   (iii) ANZAHL DER SEQUENZEN: 34
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..20
   (xi) SEQUENZBESCHREIBUNG: SEQ ID NO: 1:
   ACACCCAATT CTGAAAATGG 20
(2) INFORMATION ZU SEQ ID NO: 2:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..20
   (xi) SEQUENZBESCHREIBUNG: SEQ ID NO: 2:
   AGGTCCCTGT TCGGGCGCCA 20
(2) INFORMATION ZU SEQ ID NO: 3:
   (i) SEQUENZ CHARAKTERISTIKA:
      (A) LÄNGE: 28 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..28
   (xi) SEQUENZBESCHREIBUNG: SEQ ID NO: 3:
   GTCGACACCC AATTCTGAAA ATGGATAA 28
(2) INFORMATION ZU SEQ ID NO: 4:
   (i) SEQUENZ CHARAKTERISTIKA:
      (A) LÄNGE: 25 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..25
   (xi) SEQUENZBESCHREIBUNG: SEQ ID NO: 4:
   GCTATGTCGA CACCCAATTC TGAAA 25
(2) INFORMATION ZU SEQ ID NO: 5:
   (i) SEQUENZ CHARAKTERISTIKA:
      (A) LÄNGE: 26 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..26
   (xi) SEQUENZBESCHREIBUNG: SEQ ID NO: 5:
   GTCGCTGTCT CCGCTTCTTC TTCCTG 26
(2) INFORMATION ZU SEQ ID NO: 6:
   (i) SEQUENZ CHARAKTERISTIKA:
      (A) LÄNGE: 27 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..27
   (xi) SEQUENZBESCHREIBUNG: SEQ ID NO: 6:
   GTCTCCGCTT CTTCTTCCTG CCATAGG 27
(2) INFORMATION ZU SEQ ID NO: 7:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..20
   (xi) SEQUENZBESCHREIBUNG: SEQ ID NO: 7:
   GCGGGGCTCC ATGGGGGTCG                                                      20
(2) INFORMATION ZU SEQ ID NO: 8:
   (i) SEQUENZ CHARAKTERISTIKA:
      (A) LÄNGE: 15 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..15
   (xi) SEQUENZBESCHREIBUNG: SEQ ID NO: 8:
   CAGCTGCAAC CCAGC                                                           15
(2) INFORMATION ZU SEQ ID NO: 9:
   (i) SEQUENZ CHARAKTERISTIKA:
      (A) LÄNGE: 21 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..21
   (xi) SEQUENZBESCHREIBUNG: SEQ ID NO: 9:
   GGCTGCTGGA GCGGGGCACA C                                            21
(2) INFORMATION ZU SEQ ID NO: 10:
   (i) SEQUENZ CHARAKTERISTIKA:
      (A) LÄNGE: 15 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..15
   (xi) SEQUENZBESCHREIBUNG: SEQ ID NO: 10:
   AACGTTGAGG GGCAT                                                   15
(2) INFORMATION ZU SEQ ID NO: 11:
   (i) SEQUENZ CHARAKTERISTIKA:
      (A) LÄNGE: 18 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..18
   (xi) SEQUENZBESCHREIBUNG: SEQ ID NO: 11:
   GTGCCGGGGT CTTCGGGC                                                18
(2) INFORMATION ZU SEQ ID NO: 12:
   (i) SEQUENZ CHARAKTERISTIKA:
      (A) LÄNGE: 21 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..21
   (xi) SEQUENZBESCHREIBUNG: SEQ ID NO: 12:
   GGAGAACATC ATGGTCGAAA G                                                  21
(2) INFORMATION ZU SEQ ID NO: 13:
   (i) SEQUENZ CHARAKTERISTIKA:
      (A) LÄNGE: 22 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..22
   (xi) SEQUENZBESCHREIBUNG: SEQ ID NO: 13:
   CCCGAGAACA TCATGGTCGA AG                                                  22
(2) INFORMATION ZU SEQ ID NO: 14:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..20
   (xi) SEQUENZBESCHREIBUNG: SEQ ID NO: 14:
   GGGGAAAGCC CGGCAAGGGG                                                     20
(2) INFORMATION ZU SEQ ID NO: 15:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..20
   (xi) SEQUENZBESCHREIBUNG: SEQ ID NO: 15:
   CACCCGCCTT GGCCTCCCAC 20
(2) INFORMATION ZU SEQ ID NO: 16:
   (i) SEQUENZ CHARAKTERISTIKA:
      (A) LÄNGE: 18 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..18
   (xi) SEQUENZBESCHREIBUNG: SEQ ID NO: 16:
   GGGACTCCGG CGCAGCGC 18
(2) INFORMATION ZU SEQ ID NO: 17:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..20
   (xi) SEQUENZBESCHREIBUNG: SEQ ID NO: 17:
   GGCAAACTTT CTTTTCCTCC 20
(2) INFORMATION ZU SEQ ID NO: 18:
   (i) SEQUENZ CHARAKTERISTIKA:
      (A) LÄNGE: 19 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..19
   (xi) SEQUENZBESCHREIBUNG: SEQ ID NO: 18:
   GGGAAGGAGG AGGATGAGG                                                  19
(2) INFORMATION ZU SEQ ID NO: 19:
   (i) SEQUENZ CHARAKTERISTIKA:
      (A) LÄNGE: 21 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..21
   (xi) SEQUENZBESCHREIBUNG: SEQ ID NO: 19:
   GGCAGTCATC CAGCTTCGGA G                                               21
(2) INFORMATION ZU SEQ ID NO: 20:
   (i) SEQUENZ CHARAKTERISTIKA:
      (A) LÄNGE: 18 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..18
   (xi) SEQUENZBESCHREIBUNG: SEQ ID NO: 20:
   GCAGTAAGCA TCCATATC 18
(2) INFORMATION ZU SEQ ID NO: 21:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..20
   (xi) SEQUENZBESCHREIBUNG: SEQ ID NO: 21:
   CCCCCACCAC TTCCCCTCTC 20
(2) INFORMATION ZU SEQ ID NO: 22:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..20
   (xi) SEQUENZBESCHREIBUNG: SEQ ID NO: 22:
   CTCCCCCACC ACTTCCCCTC 20
(2) INFORMATION ZU SEQ ID NO: 23:
   (i) SEQUENZ CHARAKTERISTIKA:
      (A) LÄNGE: 19 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..19
   (xi) SEQUENZBESCHREIBUNG: SEQ ID NO: 23:
   GCTGGGAGCC ATAGCGAGG 19
(2) INFORMATION ZU SEQ ID NO: 24:
   (i) SEQUENZ CHARAKTERISTIKA:
      (A) LÄNGE: 21 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..21
   (xi) SEQUENZBESCHREIBUNG: SEQ ID NO: 24:
   ACTGCTGCCT CTTGTCTCAG G 21
(2) INFORMATION ZU SEQ ID NO: 25:
   (i) SEQUENZ CHARAKTERISTIKA:
      (A) LÄNGE: 22 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..22
   (xi) SEQUENZBESCHREIBUNG: SEQ ID NO: 25:
   CAATCAATGA CTTCAAGAGT TC 22
(2) INFORMATION ZU SEQ ID NO: 26:
   (i) SEQUENZ CHARAKTERISTIKA:
      (A) LÄNGE: 19 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..19
   (xi) SEQUENZBESCHREIBUNG: SEQ ID NO: 26:
   GGTCCCTGTT CGGGCGCCA 19
(2) INFORMATION ZU SEQ ID NO: 27:
   (i) SEQUENZ CHARAKTERISTIKA:
      (A) LÄNGE: 17 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..17
   (xi) SEQUENZBESCHREIBUNG: SEQ ID NO: 27:
   GTGCCGGGGT CTTCGGG 17
(2) INFORMATION ZU SEQ ID NO: 28:
   (i) SEQUENZ CHARAKTERISTIKA:
      (A) LÄNGE: 17 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..17
   (xi) SEQUENZBESCHREIBUNG: SEQ ID NO: 28:
   GGAGGATGCT GAGGAGG 17
(2) INFORMATION ZU SEQ ID NO: 29:
   (i) SEQUENZ CHARAKTERISTIKA:
      (A) LÄNGE: 14 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..14
   (xi) SEQUENZBESCHREIBUNG: SEQ ID NO: 29:
   GGAGGATGCT GAGG 14
(2) INFORMATION ZU SEQ ID NO: 30:
   (i) SEQUENZ CHARAKTERISTIKA:
      (A) LÄNGE: 19 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..19
   (xi) SEQUENZBESCHREIBUNG: SEQ ID NO: 30:
   CAGGAGGATG CTGAGGAGG 19
(2) INFORMATION ZU SEQ ID NO: 31:
   (i) SEQUENZ CHARAKTERISTIKA:
      (A) LÄNGE: 15 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..15
   (xi) SEQUENZBESCHREIBUNG: SEQ ID NO: 31:
   GGCTGCCATG GTCCC 15
(2) INFORMATION ZU SEQ ID NO: 32:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..20
   (xi) SEQUENZBESCHREIBUNG: SEQ ID NO: 32:
   TCATGGTGTC CTTTGCAGCC 20
(2) INFORMATION ZU SEQ ID NO: 33:
   (i) SEQUENZ CHARAKTERISTIKA:
      (A) LÄNGE: 18 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..18
   (xi) SEQUENZBESCHREIBUNG: SEQ ID NO: 33:
   TCATGGTGTC CTTTGCAG 18
(2) INFORMATION ZU SEQ ID NO: 34:
   (i) SEQUENZ CHARAKTERISTIKA:
      (A) LÄNGE: 16 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..16
   (xi) SEQUENZBESCHREIBUNG: SEQ ID NO: 34:
   AAGTTCATGG TTTCGG                                                    16